# EUROPEAN PATENT APPLICATION

(11) **EP 4 094 750 A2**
(43) Date of publication of application: **30.11.2022**
(21) Application number: 22167111.8
(22) Date of filing: 07.04.2022
(51) Int. Cl.: A61K 8/25, A61K 8/26, A61K 8/29, A61K 8/73, A61Q 19/00, A61Q 19/08, A61K 8/58, A61K 8/02

(54) **ORGANIC-INORGANIC COMPOSITE POWDER AND COMPOSITION INCLUDING THE SAME**

(30) Priority: 29.04.2021 KR 20210055825
(71) Applicant: Amorepacific Corporation, Seoul 04386 (KR)
(72) Inventor: YOU, Jaewon, Yongin-si, Gyeonggi-do 17074 (KR); LEE, Jijung, Yongin-si, Gyeonggi-do 17074 (KR); YI, Seung Hwan, Yongin-si, Gyeonggi-do 17074 (KR); JUNG, Hajin, Yongin-si, Gyeonggi-do 17074 (KR); PARK, Wonseok, Yongin-si, Gyeonggi-do 17074 (KR); BAEK, Heungsoo, Yongin-si, Gyeonggi-do 17074 (KR)
(74) Representative: Viering, Jentschura & Partner mbB Patent- und Rechtsanwälte

(57) **Abstract**

The present specification relates to an organic/inorganic composite powder and a composition including the same in which a coating film on a surface of an inorganic powder is not easily fall off under various conditions such as pH, temperature, physical force, etc., so that has excellent moisture retention and less transferring and allows for natural makeup.

## Description

### BACKGROUND OF THE INVENTION

### Field of the Invention

The present specification discloses an organic-inorganic composite powder and a composition including the same.

### Description of the Related Art

Recently, interest in natural makeup look is growing. There is a growing tendency to prefer makeup with natural gloss and transparency rather than artificial gloss.

At the same time, there is an increasing number of consumers who wish that the makeup will not crumble due to water or sweat without the dryness and stuffiness caused by makeup even after a long time after makeup.

In order to realize this demand, an attempt was made to simply mix inorganic powder and water-soluble moisturizer, but this attempt only slightly improved the dryness of the skin, and the degree of improvement in durability and transferring was not satisfactory.

In addition, in the case of anhydrous formulations such as powder/compact formulations, it is difficult to simply mix and use a water-soluble moisturizer.

Therefore, it is necessary to develop an inorganic powder that has excellent durability while solving the dryness problem and can be used even in anhydrous formulations such as powder/compact formulations.

### Documents of Related Art

### SUMMARY OF THE INVENTION

In one aspect of the present disclosure, it is an object of the present disclosure to provide an organic-inorganic composite powder having a coating film that is excellent in water retention and is not easily washed off by water.

In one aspect of the present disclosure, it is an object of the present disclosure to provide an organic-inorganic composite powder that has excellent moisture retention and does not cause the coating film thereof to fall off or be transferred by physical force.

In one aspect of the present disclosure, it is an object to provide an organic-inorganic composite powder having a smooth surface.

In one aspect of the present disclosure, it is an object of the present disclosure to provide an organic-inorganic composite powder capable of forming a transparent makeup layer by increasing transmittance to visible light.

In one aspect, the present specification provides an organic-inorganic composite powder including an inorganic powder; and an anionic polysaccharide coating layer on the inorganic powder.

In another aspect, the present specification provides a composition including the above-described organic-inorganic composite powder.

In one aspect, in the organic-inorganic composite powder disclosed herein, the inorganic powder is coated with the anionic polysaccharide to prevent the coating film from being easily washed off by water, thereby preventing a makeup layer from collapsing due to sweat or water.

In another aspect, it is possible to reduce the stuffiness caused by a hydrophobic material by coating the organic-inorganic composite powder with a hydrophilic material.

In still another aspect, by coating the anionic polysaccharide on the inorganic powder, it is possible to prevent only the coating film from transferring or separating.

In still another aspect, by coating the anionic polysaccharide on the inorganic powder, the surface (coating film) formed by the organic-inorganic composite powder is smooth and firm, providing a better appearance when applied to the skin and less transferring.

In still another aspect, since a visible light transmittance is increased by coating the anionic polysaccharide on the inorganic powder, it is possible to form a more transparent makeup layer.

### BRIEF DESCRIPTION OF THE DRAWINGS

FIG. 1 shows a result of observing whether an organic-inorganic composite powder is detached according to an embodiment of the present disclosure.
FIG. 2 shows a result of observing a degree of washing away in water of an organic-inorganic composite powder according to an embodiment of the present disclosure.
FIG. 3 shows a result of observing a degree of transferring an organic-inorganic composite powder according to an embodiment of the present disclosure.
FIG. 4 shows a result of observing a surface of a coating film of an organic-inorganic composite powder according to an embodiment of the present disclosure.
FIGS. 5a and 5b are graphs showing a visible light transmittance of an organic-inorganic composite powder according to an embodiment of the present disclosure.
FIG. 6 is a schematic diagram of an organic-inorganic composite powder according to an embodiment of the present disclosure.
FIG. 7 is a schematic diagram of a method for manufacturing an organic-inorganic composite powder according to an embodiment of the present disclosure.

### DETAILED DESCRIPTION OF THE INVENTION

Hereinafter, disclosed herein is described in more detail through the following examples.

### Organic-inorganic composite powder

In exemplary embodiments of the present disclosure, an organic-inorganic composite power including an inorganic powder; and an anionic polysaccharide coating layer on the inorganic powder is provided.

According to one embodiment of the present disclosure, the polysaccharide having an anion group is stably coated on the surface of the inorganic powder so that the polysaccharide having the anion group may not fall from the surface of the inorganic powder under various conditions such as pH, temperature, or physical force.

In one embodiment, the inorganic powder may be one selected from the group consisting of mica, alumina, titanium dioxide, aluminosilicate and silica.

In one embodiment, the inorganic powder may be mica, and the mica may be natural mica or synthetic mica.

In one embodiment, the anionic polysaccharide may comprise a carboxyl group (COO-) or a sulfate group (OSO₃⁻).

In one embodiment, the anionic polysaccharide may comprise one or more selected from the group consisting of hyaluronic acid, pectin, fucoidan, alginic acid, carrageenan, carboxyalkyl cellulose and salt thereof, gum arabic (gum acacia), alginic acid, sodium alginate, xanthan gum and chondroitin sulfate.

In one embodiment, the anionic polysaccharide may be hyaluronic acid.

In one embodiment, the inorganic powder may be surface-modified with a cationic silane-based compound.

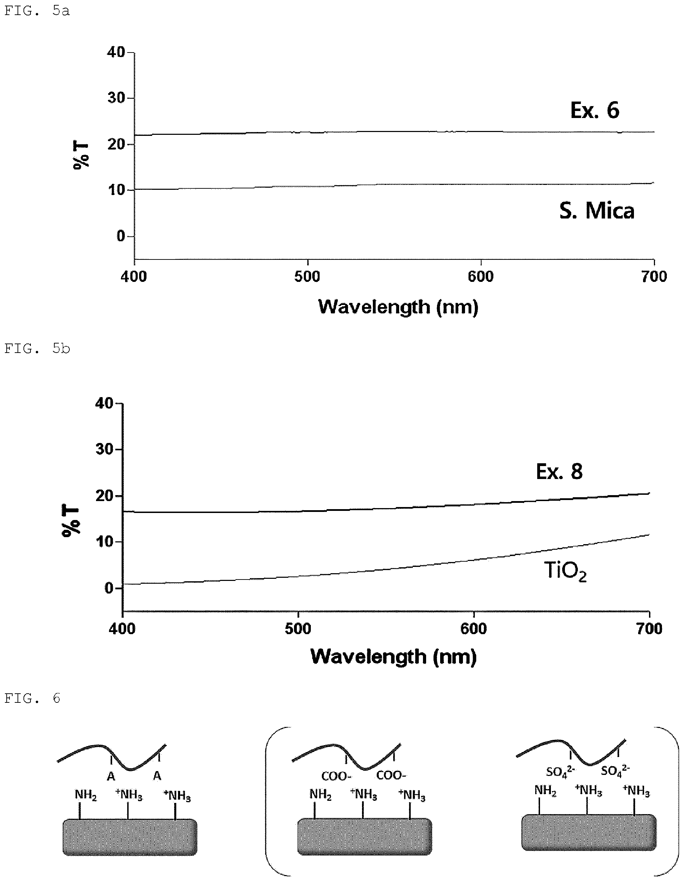

Referring to FIG. 6, the inorganic powder may have a cationic group due to the cationic silane-based compound on the surface thereof, and the inorganic powder may be in a state in which the anionic group of the anionic polysaccharide is coated on the cationic group.

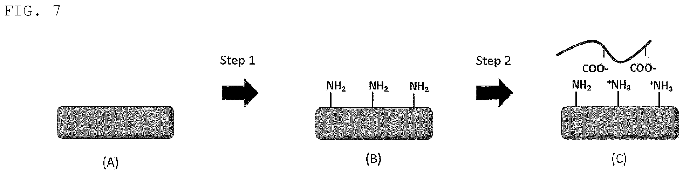

Referring to FIG. 7, a primary coating or surface modification of the inorganic powder is performed with a cationic silane-based compound capable of imparting cationic properties to the surface of the inorganic powder (Step 1), and the anionic polysaccharide is secondarily applied on the surface-modified inorganic powder (Step 2), so that a more stable coating layer can be formed.

In one embodiment, the cationic silane-based compound may comprise an amino group (NH₂) or an ammonium group (NH₃⁺).

In one embodiment, the cationic silane-based compound may be one or more selected from the group consisting of N-(2-aminoethyl)-3-aminopropylmethyldimethoxysilane, N-(2-aminoethyl)-3-aminopropyltrimethoxysilane, 3-aminopropyltrimethoxysilane, 3-aminopropyltriethoxysilane, N-phenyl-3-aminopropyltrimethoxysilane, N-(vinylbenzyl)-2-aminoethyl-3-aminopropyltrimethoxysilane hydrochloride and N-[3-(trimethoxysilyl)propyl]ethylenediamine.

In one embodiment, the cationic silane-based compound may be 3-aminopropyltriethoxysilane.

In one embodiment, the cationic silane-based compound and the anionic polysaccharide may be bonded to each other by an ionic bond.

According to one embodiment of the present disclosure, the cationic silane-based compound present on the inorganic powder binds to the anionic polysaccharide by an ionic bond, so that the embodiment of the present disclosure may form a stable coating layer maintained in various environments compared to the case in which the anionic polysaccharide is simply coated on the inorganic powder. (See FIGS. 6 and 7)

In one embodiment, the organic-inorganic composite powder may comprise 91 to 99.9 % by weight of the inorganic powder, 0.05 to 4 % by weight of the anionic polysaccharide, and 0.05 to 4 % by weight of the cationic silane-based compound, based on the total weight of the organic-inorganic composite powder, but these contents may vary depending on the type of inorganic powder.

For example, the content of the inorganic powder may be 91 % by weight or more, 92 % by weight or more, 93 % by weight or more, 94 % by weight or more, 92 % by weight or more, 96 % by weight or more, 99.9 % by weight or less, 99.8 % by weight or less, 99.7 % by weight or less, 99.6 % by weight or less, 99.5 % by weight or less, or 99.4 % by weight or less, based on the total weight of the organic-inorganic composite powder.

For example, the content of the anionic polysaccharide may be 0.05 % by weight or more, 0.1 % by weight or more, 0.15 % by weight or more, 0.2 % by weight or more, 0.25 % by weight or more, 4 % by weight or less, 3.5 % by weight or less, 3 % by weight or less, 2.5 % by weight or less, 2 % by weight or less, or 1.5 % by weight or less, based on the total weight of the organic-inorganic composite powder.

For example, the content of the cationic silane-based compound may be 0.05 % by weight or more, 0.1 % by weight or more, 0.15 % by weight or more, 0.2 % by weight or more, 0.25 % by weight or more, 4 % by weight or less, 3.5 % by weight or less, 3 % by weight or less, 2.5 % by weight or less, 2 % by weight or less, or 1.5 % by weight or less, based on the total weight of the organic-inorganic composite powder.

In one embodiment, the organic-inorganic composite powder has an improved transmittance of 10% or more, 20% or more, 30% or more, 40% or more, or 50% or more compared to the transmittance of the organic-inorganic inorganic powder not coated with the polysaccharide having an anion.

In exemplary embodiments of the present disclosure, a composition including the above-described organic-inorganic composite powder is provided.

In one embodiment, the composition may be for moisturizing.

In one embodiment, the content of the organic-inorganic composite powder may be 0.1 to 30 % by weight, for example, 0.1 % by weight or more, 0.5 % by weight or more, 1 % by weight or more, 5 % by weight or more, 10 % by weight or more, 15 % by weight or more, 20 % by weight or more, 25 % by weight or more, 30 % by weight or less, 25 % by weight or less, 20 % by weight or less, 15 % by weight or less, 10 % by weight or less, 5 % by weight or less, 1 % by weight or less, or 0.5 % by weight or less, based on the total weight of the composition.

In one embodiment, the composition may be a composition for skin external application, and the skin external preparation is a generic term that may include anything applied outside the skin, and various formulations of cosmetics and pharmaceuticals may be included therein.

In one embodiment, the composition may be a cosmetic composition.

In one embodiment, the composition may be a cosmetic composition, and the formulation of the cosmetic composition contains a cosmetically or dermatologically acceptable medium or base. This formulation includes all formulations suitable for topical application. For example, the formulation may be provided in the form of a solution, a gel, a solid, a kneaded dry product, an emulsion obtained by dispersing an oily phase in an aqueous phase, a suspension, a microemulsion, a microcapsule, a microgranule, an ionic (liposome) and non-ionic vesicular dispersant, a cream, a skin, a lotion, a powder, an ointment, a spray or a concealer stick. These compositions can be prepared according to conventional methods in the art. The composition according to the invention can also be used in the form of a foam or in the form of an aerosol composition further containing a compressed propellant.

The cosmetic composition according to an embodiment of the present disclosure may be formulated as cosmetics such as a softening lotion, an astringent lotion, a nourishing lotion, a nourishing cream, a massage cream, an essence, an eye cream, an eye essence, a cleansing Creams, a cleansing foam, a cleansing water, a cleansing tissue containing the cosmetic composition, a pack, a powder, a body lotion, a body cream, a body oil, and a body essence.

When the formulation of the present disclosure is a paste, a cream or a gel, animal fiber, vegetable fiber, wax, paraffin, starch, tracanth, cellulose derivative, polyethylene glycol, silicone, bentonite, silica, talc or zinc oxide may be used as a carrier component.

When the formulation of the present disclosure is a powder or a spray, lactose, talc, silica, aluminum hydroxide, calcium silicate or polyamide powder may be used as a carrier component, and in particular, in the case of a spray, additional propellant such as chlorofluorohydrocarbon, propane/butane or dimethyl ether may be comprised.

When the formulation of the present disclosure is a solution or an emulsion, a solvent, a solvating agent or an emulsifying agent is used as a carrier component. For example, there is water, ethanol, isopropanol, ethyl carbonate, ethyl acetate, benzyl alcohol, benzyl benzoate, propylene glycol, 1,3-butylglycol oil, glycerol fatty ester, or fatty acid ester of polyethylene glycol or sorbitan.

When the formulation of the present disclosure is a suspension, water, a liquid diluent such as ethanol or propylene glycol, a suspending agent such as ethoxylated isostearyl alcohol, polyoxyethylene sorbitol ester and polyoxyethylene sorbitan ester, microcrystalline cellulose, aluminum metahydroxide, bentonite, agar or tracanth may be used as a carrier component.

When the formulation of the present disclosure is a surfactant-containing cleansing agent, aliphatic alcohol sulfate, aliphatic alcohol ether sulfate, sulfosuccinic acid monoester, isethionate, imidazolinium derivative, methyl taurate, sarcosinate, fatty acid amide ether sulfate, alkylamidobetaine, aliphatic alcohol, fatty acid glyceride, fatty acid diethanolamide, vegetable oil, linolin derivative or ethoxylated glycerol fatty acid ester and the like may be used as a carrier component.

The cosmetic composition according to an embodiment of the present disclosure may further comprise a functional additive and components included in general cosmetic compositions in addition to the organic-inorganic composite powder. The functional additive may comprise a component selected from the group consisting of water-soluble vitamin, oil-soluble vitamin, high-molecular peptide, high-molecular polysaccharide, sphingolipid, and seaweed extract.

In the cosmetic composition of the present disclosure, a component contained in a general cosmetic composition may be further blended with the functional additive as needed. As other ingredients comprised, there may be oil and fat, a moisturizer, an emollient, a surfactant, an organic and inorganic pigment, an organic powder, a UV absorber, a preservative, a fungicide, an antioxidant, a plant extract, a pH adjuster, alcohol, a pigment, a fragrance, a blood circulation accelerator, a cooling agent, an anti-perspiration agent, purified water, etc.

The cosmetic composition according to the embodiments of the present disclosure may be a makeup composition, and the cosmetic composition may be applied to cosmetics of various formulations such as a makeup primer, a makeup base, a skin cover, a foundation, a liquid foundation, a lipstick, a lip gloss, a liquid eye shadow, an eyebrow, a concealer, a liquid concealer, a liquid blush, etc. Preferably, it can be formulated as a makeup composition such as a sponge immersion lotion, a cushion, an air cushion, and the like.

In one embodiment, the composition may be for forming a transparent makeup layer.

In one embodiment, the composition may be for forming a natural makeup layer.

For example, when makeup is performed using the above composition, a transparent makeup layer or a natural makeup layer that can give a natural feeling like the original skin color to a viewer without excessively hiding the skin color under the makeup layer can be formed.

In one embodiment, the formulation of the composition may be a foundation, a compact, or a concealer.

In another exemplary embodiment of the present disclosure, there is provided a method for improving a moisture content of a skin, including applying a composition including the above-described organic-inorganic composite powder on the skin.

In still another exemplary embodiment of the present disclosure, there is provided a use of the organic-inorganic composite powder for the preparation of a composition for moisturizing.

In still another exemplary embodiment of the present disclosure, there is provided a non-therapeutic use of a cosmetic composition including the above-described organic-inorganic composite powder for improving a moisture content of a skin.

In still another exemplary embodiment of the present disclosure, there is provided a method of forming a transparent makeup layer, including applying a composition including the above-described organic-inorganic composite powder on the skin.

In still another exemplary embodiment of the present disclosure, there is provided a use of the organic-inorganic composite powder for the preparation of a composition for forming a transparent makeup layer.

In still another exemplary embodiment of the present disclosure, there is provided a non-therapeutic use of a cosmetic composition including the above-described organic-inorganic composite powder for forming a transparent makeup layer.

Hereinafter, the present disclosure is described in more detail through the following examples.

### Embodiments

According to embodiments of the present disclosure, the organic-inorganic composite powder may be manufactured according to the following steps as shown in FIG. 7.

(Step 1) a step of coating the inorganic powder with the silane having an amino group;

(Step 2) a step of re-coating the inorganic powder with the anionic polysaccharide.

### [Preparation Example 1] Preparation of aminosilane-coated silica

50 g of silica (CL silica 500, obtained from Chemiland) is added to 500 mL of 95% ethanol and stirred. 3 mL of 3-aminopropyltriethoxysilane is slowly added dropwise to the reaction solution, the temperature of the reaction solution is raised and refluxed for 15 hours. The temperature of the reaction solution is lowed, filtered and washed with 95% EtOH. The prepared aminosilane-coated silica is dried.

### [Preparation Example 2] Preparation of aminosilane-coated silica

50 g of silica (CL silica 500, obtained from Chemiland) is calcinated at 600 °C, and then, stored in a desiccator. The silica is added to 500 mL of toluene and stirred. The reason for doing this is to completely remove moisture at a high temperature such as 600 °C, so that the coating can be performed well. 3 mL of 3-aminopropyltriethoxysilane is slowly added dropwise to the reaction solution, and the temperature of the reaction solution is raised and refluxed for 15 hours. The temperature of the reaction solution is lowered, filtered, and washed with toluene and EtOH. The prepared aminosilane-coated silica is dried.

### [Preparation Example 3] Preparation of aminosilane-coated mica

50 g of mica (MICA Y-1800, obtained from Yamaguchi Mica) is added in 500 mL of 95% ethanol and stirred. 2.5 mL of 3-aminopropyltriethoxysilane is slowly added dropwise to the reaction solution, the temperature of the reaction solution is raised and refluxed for 15 hours. The temperature of the reaction solution is lowered, filtered, and wash with 95% EtOH. The prepared aminosilane-coated mica is dried.

### [Preparation Example 4] Preparation of aminosilane-coated synthetic mica

50 g of synthetic mica (NK-8G, obtained from Korea Synthetic Pearl) is added in 500 mL of 95% ethanol and stirred. 3.0 mL of 3-aminopropyltriethoxysilane is slowly added dropwise to the reaction solution, the temperature of the reaction solution is raised and refluxed for 15 hours. The temperature of the reaction solution is lowered, filtered, and washed with 95% EtOH. The prepared aminosilane-coated synthetic mica is dried.

### [Preparation Example 5] Preparation of aminosilane-coated synthetic mica

50 g of synthetic mica (NK-8G, obtained from Korea Synthetic Pearl) is added in 500 mL of 95% ethanol and stirred. 3.0 mL of *N*-[3-(trimethoxysilyl)propyl]ethylenediamine is slowly added dropwise to the reaction solution, the temperature of the reaction solution is raised, and refluxed for 15 hours. The temperature of the reaction solution is lowered, filtered, and washed with 95% EtOH. The prepared aminosilane-coated mica is dried.

### [Preparation Example 6] Preparation of aminosilane-coated TiO₂

50 g of TiO₂ (not for sale, obtained from Korea Synthetic Pearl) is added in 500 mL of 95% ethanol and stirred. 3.0 mL of 3-aminopropyltriethoxysilane is slowly added dropwise to the reaction solution, the temperature of the reaction solution is raised and refluxed for 15 hours. The temperature of the reaction solution is lowered, filtered, and washed with 95% EtOH. The prepared aminosilane-coated TiO₂ is dried.

### [Example 1] Preparation of HA coated silica

1 g of sodium hyaluronate is added in 100 mL of distilled water while stirring, and then 400 mg of citric acid is added to the reaction solution to prepare an HA aqueous solution. 50 g of the aminosilane silica obtained in Preparation Example 1 is added in 200 g of distilled water, and then well dispersed. Then, the reaction solution is stirred while slowly adding the prepared HA aqueous solution. After additional stirring for 10 minutes, the reaction solution is filtered and washed thoroughly with water and EtOH. The prepared HA-coated silica is dried.

### [Example 2] Preparation of HA coated silica

1 g of sodium hyaluronate is added in 100 mL of distilled water while stirring, and then, 400 mg of citric acid is added to the reaction solution to prepare an HA aqueous solution. 50 g of the aminosilane-coated silica obtained in Preparation Example 2 is added in 200 g of distilled water, well dispersed, and then stirred while slowly adding the prepared HA aqueous solution. After additional stirring for 10 minutes, the reaction solution is filtered and washed thoroughly with water and EtOH. The prepared HA-coated silica is dried.

### [Example 3] Preparation of HA coated mica

500 mg of sodium hyaluronate is added in 50 mL of distilled water while stirring, and then, 100 mg of citric acid is added to prepare an HA aqueous solution. 50 g of aminosilane-coated mica obtained in Preparation Example 3 is added in 200 g of distilled water, well dispersed, and then stirred while slowly adding the previously prepared HA aqueous solution. After additional stirring for 10 minutes, the reaction solution is filtered and washed thoroughly with water and EtOH. The prepared HA-coated mica is dried.

### [Example 4] Preparation of pectin coated mica

500 mg of pectin is added in 50 mL of distilled water while stirring, and then, 100 mg of citric acid is added to the reaction solution to prepare an HA aqueous solution. 50 g of aminosilane-coated mica obtained in Preparation Example 3 is added in 200 g of distilled water, well dispersed, and then stirred while slowly adding the previously prepared HA aqueous solution. After additional stirring for 10 minutes, the reaction solution is filtered and washed thoroughly with water and EtOH. The prepared pectin-coated mica is dried.

### [Example 5] Preparation of fucoidan coated mica

500 mg of fucoidan is added in 50 mL of distilled water while stirring, and then, 100 mg of citric acid is added to the reaction solution to prepare an HA aqueous solution. 50 g of aminosilane-coated mica obtained in Preparation Example 3 is added in 200 g of distilled water, well dispersed, and then stirred while slowly adding the previously prepared HA aqueous solution. After additional stirring for 10 minutes, the reaction solution is filtered and washed thoroughly with water and EtOH. The prepared fucoidan-coated mica is dried.

### [Example 6] Preparation of HA coated synthetic mica

500 mg of sodium hyaluronate is added in 50 mL of distilled water while stirring, and then, 150 mg of citric acid is added to the reaction solution to prepare an HA aqueous solution. 50 g of the aminosilane-coated synthetic mica obtained in Preparation Example 4 is added in 200 g of distilled water, well dispersed, and then stirred while slowly adding the prepared HA aqueous solution. After additional stirring for 10 minutes, the reaction solution is filtered and washed thoroughly with water and EtOH. The prepared HA-coated synthetic mica is dried.

### [Example 7] Preparation of HA coated synthetic mica

1 g of sodium hyaluronate is added in 50 mL of distilled water while stirring, and then, 300 mg of citric acid is added to the reaction solution to prepare an HA aqueous solution. 50 g of the aminosilane-coated synthetic mica obtained in Preparation Example 5 is added in 200 g of distilled water, well dispersed, and then stirred while slowly adding the previously prepared HA aqueous solution. After additional stirring for 10 minutes, the reaction solution is filtered and washed thoroughly with water and EtOH. The prepared HA-coated synthetic mica is dried.

### [Example 8] Preparation of HA coated TiO₂

500 mg of sodium hyaluronate is added in 50 mL of distilled water while stirring, and then, 100 mg of citric acid is added to the reaction solution to prepare an HA aqueous solution. 50 g of aminosilane-coated TiO₂ obtained in Preparation Example 6 is added in 200 g of distilled water, well dispersed, and then stirred while slowly adding the previously prepared HA aqueous solution. After additional stirring for 10 minutes, the reaction solution is filtered and washed thoroughly with water and EtOH. The prepared HA coated TiO₂ is dried.

### [Comparative Example 1]

500 mg of sodium hyaluronate is added in 50 mL of distilled water while stirring, and then 100 mg of citric acid is added to the reaction solution to prepare an HA aqueous solution. 50 g of synthetic mica is added in 200 g of distilled water, well dispersed, and then stirred while slowly adding the previously prepared HA aqueous solution. After additional stirring for 10 minutes, the reaction solution is filtered and washed thoroughly with water and EtOH. The filtered powder is dried.

### [Comparative Example 2]

16.5 mg of sodium hyaluronate is added in 50 mL of distilled water while stirring, and then 5 mg of citric acid is added to the reaction solution to prepare an HA aqueous solution. 50 g of synthetic mica is added in the aqueous HA solution, stirred, well dispersed, and then dried at 105 °C.

### [Experimental Example 1] Measurement of loss on ignition

In order to determine the coating amount of the prepared sample powder, the ignition residue was measured. After weighing the sufficiently dried container, about 10 g of the sample was put in the container and the weight was measured. After heating the sample contained in the container at 600 °C for 6 hours in a furnace, the temperature of the sample was lowered in a desiccator and the sample was dried sufficiently. Then, the weight was measured to determine the percent weight loss relative to the sample weight. The coating amount of the anionic polysaccharide was determined from the difference between the value of the preparation example coated with the respective corresponding aminosilane and the example coated with the anionic polysaccharide.

As shown in Table 1 below, it can be confirmed that about 0.2 % to 2 % of the coating was applied. In the case of Comparative Example 1, in which the anionic polysaccharide was directly coated without aminosilane coating, the coating amount of the anionic polysaccharide was determined from the difference between the synthetic mica, which was the inorganic powder used for coating, and the comparative example. As a result, it can be seen that there is almost no coating of the anionic polysaccharide.

**[Table 1]**

| Classification | Loss on ignition (%) | Coating amount of anionic polysaccharide (%) |
|---|---|---|
| Preparation Example 1 | 4.61 | Not applicable |
| Preparation Example 2 | 4.79 | Not applicable |
| Preparation Example 3 | 3.13 | Not applicable |
| Preparation Example 4 | 0.33 | Not applicable |
| Preparation Example 5 | 0.37 | Not applicable |
| Preparation Example 6 | 1.48 | Not applicable |
| Example 1 | 5.97 | 1.36 |
| Example 2 | 6.33 | 1.54 |
| Example 3 | 3.68 | 0.55 |
| Example 4 | 3.86 | 0.73 |
| Example 5 | 3.56 | 0.43 |
| Example 6 | 0.65 | 0.32 |
| Example 7 | 0.70 | 0.33 |
| Example 8 | 2.05 | 0.57 |
| Comparative Example 1 | 0.19 | 0.01 |
| Synthetic mica | 0.18 | Not applicable |

### [Experimental Example 2] Measurement of zeta potential

In order to check the presence or absence of coating of the prepared sample powder and the surface charge characteristics of the powder, zeta potential was measured using a DLS measuring instrument, and the results are shown in a table.

As shown in Table 2 below, it can be seen that the surface charge characteristics changed through the aminosilane coating and the anionic polysaccharide coating. In addition, it can be seen that Comparative Example 1, which showed almost no anionic polysaccharide coating amount in Experimental Example 1, had different surface charge characteristics from those of Examples 6 or 7 coated with the anionic polysaccharide.

**[Table 2]**

| Classification | Zeta potential (mV) |
|---|---|
| Silica | -33.5 |
| Mica | -34.0 |
| Synthetic mica | -41.8 |
| TiO₂ | +10.9 |
| Preparation Example 1 | +11.0 |
| Preparation Example 2 | +13.2 |
| Preparation Example 3 | +31.1 |
| Preparation Example 4 | +36.7 |
| Preparation Example 5 | +44.1 |
| Preparation Example 6 | +28.2 |
| Example 1 | +2.0 |
| Example 2 | -0.1 |
| Example 3 | +0.1 |
| Example 4 | -21.8 |
| Example 5 | -27.1 |
| Example 6 | -1.22 |
| Example 7 | -12.6 |
| Example 8 | +13.4 |
| Comparative Example 1 | -41.9 |

### [Experimental Example 3] Determination of desorption of coated anionic polysaccharides

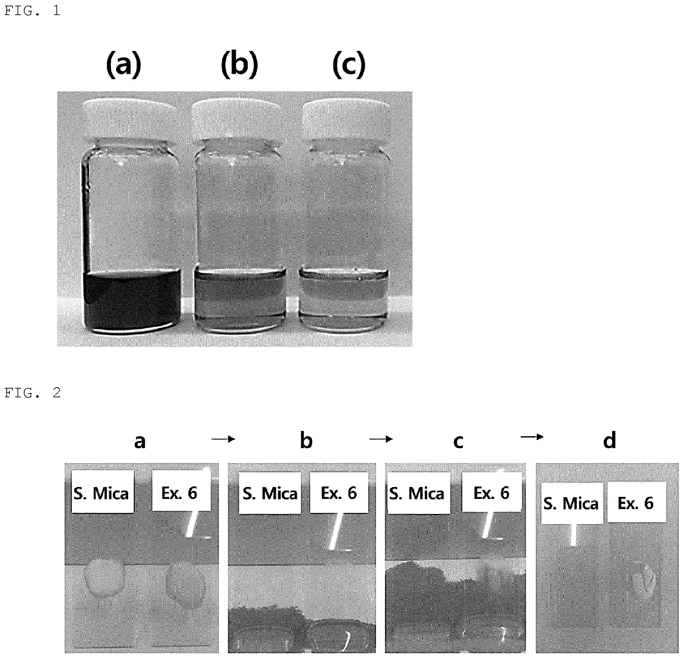

In order to determine whether the anionic polysaccharide coated on the prepared sample powder is desorbed in an aqueous solution according to pH and temperature conditions, each of the hyaluronic acid samples corresponding to 1 % by weight, 10 % by weight, and 100 % by weight of the hyaluronic acid coating amount of Comparative Example 2 and the sample of Example 6 was stirred for 10 minutes in an aqueous solution of specific pH and temperature conditions, and filtered. The amount of desorbed hyaluronic acid in the filtered solution was determined using carbazole assay, a method for detecting hyaluronic acid. 0.95 g of borax and 100g of sulfuric acid were mixed and stirred slowly for 48 to 72 hours to prepare a borax solution. 0.125 g of carbazole was added in 100 ml of anhydrous ethanol and stirred to prepare a carbazole solution. 5 mL of the borax solution was put into a vial installed in an ice bath, and 1 mL of the measurement sample was added to the vial and stirred. The vial was transferred to a bath set at 98 °C and stirred for 15 minutes. After cooling again in the ice bath, 0.2 mL of the carbazole solution was added to the vial and stirred. Further, the vial was transferred to the bath set at 98 °C and stirred for 15 minutes. Then, the colored solution was cooled to room temperature. The hyaluronic acids corresponding to 1 % by weight, 10 % by weight, and 100 % by weight of the hyaluronic acid coating amount of the sample of Example 6 were tested and its test result was shown in FIG. 1. In FIG. 1, (a) shows 100% desorption, (b) shows 10% desorption, and (c) shows 1% desorption.

Based on this result, the degree of desorption of the coated hyaluronic acid was determined as shown in Table 3 and shown in Table 4.

Specifically, in the table of Experimental Example 1, the coating amount of the anionic polysaccharide of Example 6 was 0.32 % by weight, and in order to make a comparison sample to determine whether the coating was desorbed, assuming that 1 % by weight, 10 % by weight, 100 % by weight of the coating amount (0.32 % by weight) of Example 6 were desorbed, the carbazole assay was performed with 0.0032 % by weight, 0.032 % by weight, and 0.32 % by weight of HA (not HA-coated inorganic powder) corresponding to the above assumption, respectively. In addition, 'the color of the comparative sample subjected to the carbazole assay' and the 'color of the HA-coated inorganic powder subjected to the carbazole assay under each condition' were compared and evaluated as shown in Table 3. The sample corresponding to 100 % was not the same sample as Example 6, only the amount of HA contained in the sample was the same as the sample of Example 6.

Referring to Tables 3 and 4 below, in the case of Example 6, the coating was not desorbed but in a stable state at room temperature and 50 °C. Some desorption was observed at a high temperature of 70 °C or higher, but it was confirmed that most of the coating was rarely desorbed. In addition, in the case of Comparative Example 2, in which hyaluronic acid was simply physically covered for coating, it can be seen that the coated hyaluronic acid was easily desorbed under all conditions.

**[Table 3]**

| Desorption amount | Within 1 % | 1 to 10 % | 10 % or greater |
|---|---|---|---|
| Result of determination | Very good | Good | Poor |

**[Table 4]**

| Classification | Condition | Result of determination |
|---|---|---|
| Example 6 | Room temperature | Very good |
| Example 6 | 50 °C | Very good |
| Example 6 | 70 °C | Good |
| Comparative Example 2 | Room temperature | Poor |
| Comparative Example 2 | 50 °C | Poor |
| Comparative Example 2 | 70 °C | Poor |

### [Experimental Example 4] Moisture retention test

A drying chamber was prepared by adding calcium chloride for drying into a sealed chamber. The measurement sample was thoroughly mixed with 100 to 200 % of the same mass of water, and then the mass was measured. After storage for 4 days in the drying chamber, the mass was measured. From the measured mass, the ratio of the amount of remaining moisture to the initial amount of moisture was calculated as a percentage to calculate the water retention rate, and was shown in Table 5. As a comparative group, the inorganic powder that was not coated or the inorganic powder that was not coated with the polysaccharide having an anion but was simply mixed, instead of in the Example, was also measured. In this case, the simply mixed polysaccharide having an anion was used in the same amount as the coating amount of the anionic polysaccharide of each Example obtained in Experimental Example 1.

As shown in Table 5 below, it was confirmed that the moisture retaining was superior to that of the uncoated inorganic powder or the inorganic powder simply mixed without being coated with the polysaccharide having anion.

**[Table 5]**

| Classification | Moisture retention rate (%) |
|---|---|
| Mica | 24 % |
| Simple mix of mica and pectin | 31 % |
| Example 4 | 34 % |
| Synthetic mica | 25 % |
| Simple mix of synthetic mica and HA | 27 % |
| Example 6 | 29 % |
| TiO₂ | 47 % |
| Simple mix of TiO₂ and HA | 50 % |
| Example 8 | 53 % |

### [Experimental Example 5] Degree of washout by water

1g of the measurement sample was well dispersed in 2mL of water, and then 50 µL of the dispersion was taken and dropped on a slide glass and dried sufficiently at room temperature to form a coating film. In FIG. 2, (a) shows the observation while slowly dropping 20 mL of water after placing the slide glass on an inclined place. (b, c) show the observation again after all the water had been drained and the water was removed, (d) shows the results of this experiment. Here, (a) shows a coating film formation stage, (b) shows an initial stage of water draining, (c) shows a late stage of water draining, and (d) shows a state after water removal. In addition, the appearance of synthetic mica (indicated as S. Mica in the drawing) and Example 6 (indicated as Ex. 6 in the drawing) are shown, respectively. Table 6 shows the results determined based on the amount of powder remained after water removal.

As a result of observation, as shown in Table 6 below, it can be seen that the coating film is less washed out by water compared to the inorganic powder not coated with anionic polysaccharides, which is considered to have an effect of preventing the makeup layer from collapsing by sweat or water. In particular, it remains less washed out by water despite being coated with a hydrophilic material rather than a hydrophobic material, which is expected to reduce the stuffiness caused by the hydrophobic material.

**[Table 6]**

| Classification | Degree of washout by water |
|---|---|
| Mica | Very high |
| Synthetic mica | Very high |
| Example 3 | Low |
| Example 4 | Low |
| Example 6 | Low |
| Example 7 | Low |

### [Experimental Example 6] Improvement test of powder transferring

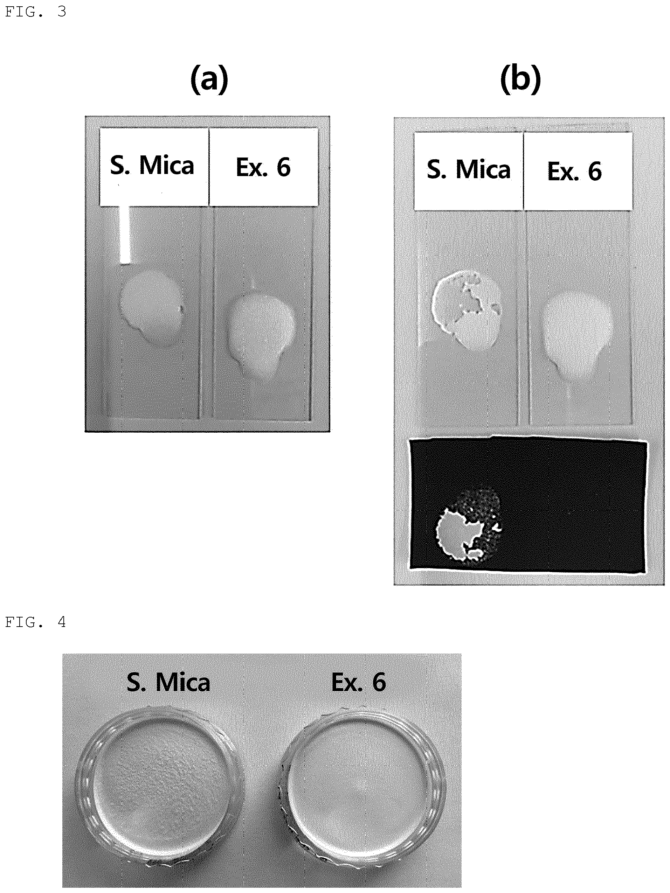

1 g of the measurement sample was dispersed in 2 mL of water, and then 50 µL of the dispersion was taken and dropped on a slide glass, and then sufficiently dried at room temperature. A black cloth or artificial leather was placed on the dried coating film, and an object of a certain weight was placed on it and leaved for 1 minute. The amount of powder attached to the black cloth was observed as shown in FIG. 3. In FIG. 3, (a) shows an image before placing the object of a certain weight on the black cloth, (b) shows an image confirming the degree of transferring on the black cloth after the object of a certain weight was placed on the black cloth. In addition, the appearance of synthetic mica (indicated as S. Mica in the drawing) and Example 6 (indicated as Ex. 6 in the drawing) were shown, respectively.

Table 7 shows the results of determination based on the transferred amount.

As a result of observation, it was confirmed that transferring was reduced compared to the non-coated one, by stably coating the anionic polysaccharide on the surface of the inorganic powder.

**[Table 7]**

| Classification | Degree of transferring |
|---|---|
| Mica | High |
| Synthetic mica | High |
| Example 3 | Very low |
| Example 4 | Very low |
| Example 6 | Very low |
| Example 7 | Very low |

### [Experimental Example 7] Formation of a smooth coating film

1g of the measurement sample was well dispersed in 2mL of water, placed in a petri dish, and sufficiently dried at room temperature. The smoothness of the coating film formed after drying was visually observed as shown in FIG. 4. Then, the gloss of this coating film was measured using a gloss meter and shown in Table 8. FIG. 4 shows the appearance of synthetic mica (indicated as S. Mica in the drawing) and Example 6 (indicated as Ex. 6 in the drawing).

As a result of observation, it was found that the sample coated with the anionic polysaccharide was smoother with the naked eye compared to the sample that was not coated with the anionic polysaccharide, and the gloss value was higher due to the smooth surface.

**[Table 8]**

| Classification | Gloss |
|---|---|
| Mica | 112.5 |
| Synthetic mica | 291.8 |
| Example 3 | 121.9 |
| Example 6 | 327.5 |
| Example 7 | 326.1 |

### [Experimental Example 8] Transmittance measurement for visible light

After the measurement sample was dispersed in distilled water at a concentration of 0.001 to 0.01 % by weight, the degree of transmission for visible light in a wavelength region of 400 to 700 nm was measured using a UV/VIS instrument. Specifically, it was measured with the synthetic mica at a concentration of 0.01 % by weight, and the titanium at a concentration of 0.001 % by weight. FIG. 5a shows the graph of synthetic mica (indicated as S. Mica in the drawing) and Example 6 (indicated as Ex. 6 in the drawing), respectively. In addition, FIG. 5b shows the graph of titanium (indicated as TiO₂ in the drawing) and Example 8 (indicated as Ex. 8 in the drawing), respectively.

As shown in FIGS. 5a and 5b, it was confirmed that the sample coated with the anionic polysaccharide had higher transmittance for visible light in the wavelength region of 400 to 700 nm compared to the sample on which the anionic polysaccharide was not coated. Due to the increased transmittance to visible light, it can be expected to form a more transparent makeup layer.

### Exemplary embodiments

Exemplary embodiment 1: an organic-inorganic composite powder including an inorganic powder and an anionic polysaccharide coating layer on the inorganic powder.

Exemplary embodiment 2: The organic-inorganic composite powder of the exemplary embodiment 1, wherein the inorganic powder is one selected from the group consisting of mica, alumina, titanium dioxide, aluminosilicate and silica.

Exemplary embodiment 3: The organic-inorganic composite powder according to the exemplary embodiment 1 or 2, wherein the inorganic powder is mica.

Exemplary embodiment 4: The organic-inorganic composite powder according to any one of the exemplary embodiments 1 to 3, wherein the anionic polysaccharide has a carboxyl group (COO-) or a sulfate group (OSO₃⁻).

Exemplary embodiment 5: The organic-inorganic composite powder according to any one of the exemplary embodiments 1 to 4, wherein the anionic polysaccharide comprises one or more selected from the group consisting of hyaluronic acid, pectin, fucoidan, alginic acid, carrageenan, carboxyalkyl cellulose and salt thereof, gum arabic (gum acacia), alginic acid, sodium alginate, xanthan gum and chondroitin sulfate.

Exemplary embodiment 6: The organic-inorganic composite powder according to any one of the exemplary embodiments 1 to 5, wherein the anionic polysaccharide is the hyaluronic acid.

Exemplary embodiment 7: The organic-inorganic composite powder according to any one of the exemplary embodiments 1 to 6, wherein the content of the anionic polysaccharide is 0.05 to 4 % by weight based on the total weight of the organic-inorganic composite powder.

Exemplary embodiment 8: The organic-inorganic composite powder according to any one of the exemplary embodiments 1 to 7, wherein the inorganic powder is surface-modified with a cationic silane-based compound.

Exemplary embodiment 9: The organic-inorganic composite powder according to the exemplary embodiment 8, wherein the cationic silane-based compound comprises an amino group (NH₂) or an ammonium group (NH₃⁺).

Exemplary embodiment 10: The organic-inorganic composite powder according to the exemplary embodiment 8 or 9, wherein the cationic silane-based compound comprises one or more selected from the group consisting of N-(2-aminoethyl)-3-aminopropylmethyldimethoxysilane, N-(2-aminoethyl)-3-aminopropyltrimethoxysilane, 3-aminopropyltrimethoxysilane, 3-aminopropyltriethoxysilane, N-phenyl-3-aminopropyltrimethoxysilane, N-(vinylbenzyl)-2-aminoethyl-3-aminopropyltrimethoxysilane hydrochloride and N-[3-(trimethoxysilyl)propyl]ethylenediamine.

Exemplary embodiment 11: The organic-inorganic composite powder according to any one of the exemplary embodiments 8 to 10, wherein the cationic silane-based compound is 3-aminopropyltriethoxysilane.

Exemplary embodiment 12: The organic-inorganic composite powder according to any one of the exemplary embodiments 8 to 11, wherein the cationic silane-based compound and the anionic polysaccharide are bonded to each other by an ionic bond.

Exemplary embodiment 13: The organic-inorganic composite powder according to any one of the exemplary embodiments 8 to 12, wherein based on the total weight of the organic-inorganic composite powder, 91 to 99.9 % by weight of the inorganic powder, 0.05 to 4 % by weight of the anionic polysaccharide, and 0.05 to 4 % by weight of the cationic silane-based compound are comprised.

Exemplary embodiment 14: A composition including the organic-inorganic composite powder according to any one of the exemplary embodiments 1 to 13.

Exemplary embodiment 15: The composition according to the exemplary embodiment 14, wherein the content of the organic-inorganic composite powder is 0.1 to 30 % by weight based on the total weight of the composition.

Exemplary embodiment 16: The composition according to the exemplary embodiment 14 or 15, wherein the composition is for moisturizing.

Exemplary embodiment 17: The composition according to any one of the exemplary embodiments 14 to 16, wherein the composition is a composition for skin external application.

Exemplary embodiment 18: The composition according to any one of the exemplary embodiments 14 to 17, wherein the composition is a cosmetic composition.

Exemplary embodiment 19: The composition according to any one of the exemplary embodiments 14 to 18, wherein the composition is for forming a transparent makeup layer.

Exemplary embodiment 20: The composition according to any one of the exemplary embodiments 14 to 19, wherein a formulation of the composition is a foundation, a compact or a concealer.

## Claims

1. An organic-inorganic composite powder, comprising:
an inorganic powder; and
an anionic polysaccharide coating layer on the inorganic powder.

2. The organic-inorganic composite powder according to claim 1, wherein the inorganic powder is one selected from the group consisting of mica, alumina, titanium dioxide, aluminosilicate and silica, preferably mica.

3. The organic-inorganic composite powder according to claim 1 or 2, wherein the anionic polysaccharide comprises a carboxyl group (COO-) or a sulfate group (OSO₃⁻), preferably comprises one or more selected from the group consisting of hyaluronic acid, pectin, fucoidan, alginic acid, carrageenan, carboxyalkyl cellulose and salt thereof, gum arabic (gum acacia), alginic acid, sodium alginate, xanthan gum and chondroitin sulfate, more preferably comprises hyaluronic acid.

4. The organic-inorganic composite powder according to any one of claims 1 to 3, wherein a content of the anionic polysaccharide is 0.05 to 4 % by weight based on a total weight of the organic-inorganic composite powder.

5. The organic-inorganic composite powder according to any one of claims 1 to 4, wherein the inorganic powder is surface-modified with a cationic silane-based compound.

6. The organic-inorganic composite powder according to claim 5, wherein the cationic silane-based compound comprises an amino group (NH₂) or an ammonium group (NH₃⁺), preferably comprises one or more selected from the group consisting of N-(2-aminoethyl)-3-aminopropylmethyldimethoxysilane, N-(2-aminoethyl)-3-aminopropyltrimethoxysilane, 3-aminopropyltrimethoxysilane, 3-aminopropyltriethoxysilane, N-phenyl-3-aminopropyltrimethoxysilane, N-(vinylbenzyl)-2-aminoethyl-3-aminopropyltrimethoxysilane hydrochloride and N-[3-(trimethoxysilyl)propyl]ethylenediamine, more preferably comprises 3-aminopropyltriethoxysilane.

7. The organic-inorganic composite powder according to claim 5, wherein the cationic silane-based compound and the anionic polysaccharide are bonded to each other by an ionic bond.

8. The organic-inorganic composite powder according to claim 5, wherein based on a total weight of the organic-inorganic composite powder, 91 to 99.9 % by weight of the inorganic powder, 0.05 to 4 % by weight of the anionic polysaccharide, and 0.05 to 4 % by weight of the cationic silane-based compound are comprised.

9. A composition comprising the organic-inorganic composite powder according to any one of claims 1 to 8.

10. The composition according to claim 9, wherein the content of the organic-inorganic composite powder is 0.1 to 30 % by weight based on a total weight of the composition.

11. The composition according to claim 9, wherein the composition is for skin external application.

12. The composition according to claim 9, wherein the composition is a cosmetic composition.

13. The composition according to claim 12, wherein the composition is for forming a transparent makeup layer on skin.

14. The composition according to claim 12, wherein a formulation of the composition is a foundation, a compact or a concealer.

15. Use of the composition according to claim 9 for skin moisturizing.
